# NEW EUROPEAN PATENT SPECIFICATION

(11) **EP 3 078 667 B2**
(45) Date of publication and mention of the opposition decision: **14.10.2026**
(45) Mention of the grant of the patent: 21.11.2018
(21) Application number: 16157001.5
(22) Date of filing: 25.01.2002
(51) Int. Cl.: C07F 5/02, C07F 5/04, C07K 5/078, A61K 38/05, A61K 31/69, A61P 35/00, A61P 29/00

(54) **METHOD OF PREPARING A LYOPHILIZED COMPOUND**
VERFAHREN ZUR HERSTELLUNG EINER LYOPHILISIERTEN VERBINDUNG
PROCÉDÉ DE PRÉPARATION D'UN COMPOSÉ LYOPHILISÉ

(30) Priority: 25.01.2001 US 264160 P
(43) Date of publication of application: 12.10.2016
(62) Document number(s) of the earlier application(s) in accordance with Art. 76 EPC: 10006835.2 / 2 251 344; 02709145.3 / 1 355 910
(73) Proprietor: The United States of America, represented by the Secretary, Department of Health and Human Services, Rockville, MD 20852 (US)
(72) Inventor: GUPTA, Shanker, Rockville, MD Maryland 20850 (US)
(74) Representative: Carpmaels & Ransford LLP

(56) References cited:
- WO-A1-96/13266
- WU S ET AL: "DEGRADATION PATHWAYS OF A PEPTIDE BORONIC ACID DERIVATIVE, 2-PYZ-(CO)-PHE-LEU-B(OH)2", JOURNAL OF PHARMACEUTICAL SCIENCES, AMERICAN PHARMACEUTICAL ASSOCIATION, WASHINGTON, US, vol. 89, no. 6, 1 June 2000 (2000-06-01), pages 758 - 765, XP001197158, ISSN: 0022-3549, DOI: 10.1002/(SICI)1520-6017(200006)89:6<758::AID-JPS7>3.0.CO;2-L
- ALEXANDRA I. KIM ET AL: "The Physical State of Mannitol after Freeze-Drying: Effects of Mannitol Concentration, Freezing Rate, and a Noncrystallizing Cosolute", JOURNAL OF PHARMACEUTICAL SCIENCES, vol. 87, no. 8, 1 August 1998 (1998-08-01), WASHINGTON, US, pages 931 - 935, XP055248890, ISSN: 0022-3549, DOI: 10.1021/js980001d

## Description

### FIELD OF THE INVENTION

This invention relates to the formulation of pharmaceutical compounds. More particularly, the invention relates to stable, pharmaceutically acceptable compositions prepared from boronic acid compounds. The invention also relates to methods for preparing such compositions.

### BACKGROUND OF THE INVENTION

Boronic acid and ester compounds display a variety of pharmaceutically useful biological activities. Shenvi et al., U.S. Pat. No. 4,499,082 (1985), discloses that peptide boronic acids are inhibitors of certain proteolytic enzymes. Kettner and Shenvi, U.S. Pat. No. 5,187,157 (1993); U.S. Pat. No. 5,242,904 (1993); and U.S. Pat. No. 5,250,720 (1993), describe a class of peptide boronic acids that inhibit trypsin-like proteases. Kleeman et al., U.S. Pat. No. 5,169,841 (1992), discloses N-terminally modified peptide boronic acids that inhibit the action of renin. Kinder et al., U.S. Pat. No. 5,106,948 (1992), discloses that certain tripeptide boronic acid compounds inhibit the growth of cancer cells.

Adams et al., U.S. Patent No. 5,780,454 (1998), U.S. Patent No. 6;066,730 (2000), U.S. Patent No. 6,083,903 (2000), and U.S. Patent No. 6,297,217 (2001), hereby incorporated by reference in their entirety, describe peptide boronic ester and acid compounds useful as proteasome inhibitors. The references also describe the use of boronic ester and acid compounds to reduce the rate of muscle protein degradation, to reduce the activity of NF-κB in a cell, to reduce the rate of degradation of p53 protein in a cell, to inhibit cyclin degradation in a cell, to inhibit the growth of a cancer cell, to inhibit antigen presentation in a cell, to inhibit NF-κB dependent cell adhesion, and to inhibit HIV replication. Brand et al., WO 98/35691, teaches that proteasome inhibitors, including boronic acid compounds, are useful for treating infarcts such as those that occur during stroke or myocardial infarction. Elliott et al., WO 99/15183, teaches that proteasome inhibitors are useful for treating inflammatory and autoimmune diseases.

Unfortunately, alkylboronic acids are relatively difficult to obtain in analytically pure form. Snyder et al., J. Am. Chem. Soc., 3611 (1958), teaches that alkylboronic acid compounds readily form boroxines (anhydrides)under dehydrating conditions. Also, alkylboronic acids and their boroxines are often air-sensitive. Korcek et al., J. Chem. Soc., Perkin Trans. 2 242 (1972), teaches that butylboronic acid is readily oxidized by air to generate 1-butanol and boric acid. These difficulties limit the pharmaceutical utility of boronic acid compounds, complicating the characterization of pharmaceutical agents comprising boronic acid compounds and limiting their shelf life.

There is thus a need in the art for improved formulations of boronic acid compounds. Ideally, such formulations would be conveniently prepared, would exhibit enhanced stability and longer shelf life as compared to the free boronic acid compound, and would readily liberate the bioactive boronic acid compound when administered to a subject in need of boronic acid therapy.

### SUMMARY

As described in claims 1 and 2 and strictly limited to the content of these claims the present invention provides stable, pharmaceutically acceptable compositions prepared from boronic acid compounds. The invention also provides methods for preparing such compositions. The invention provides the discovery that lyophilization of an aqueous mixture comprising a boronic acid compound and a compound having at least two hydroxyl groups produces a stable composition that readily releases the boronic acid compound upon dissolution in aqueous media.

Disclosed are generally compounds having formula (1): 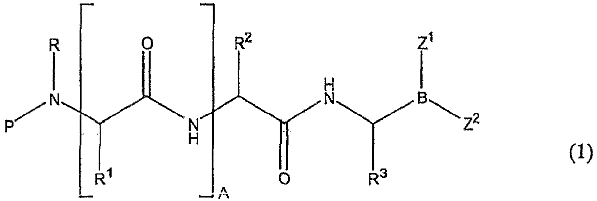 wherein:
P is hydrogen or an amino-group protecting moiety;
R is hydrogen or alkyl;
A is 0, 1, or 2;
R¹, R², and R³ are independently hydrogen, alkyl, cycloalkyl, aryl, or -CH₂-R⁵;
R⁵, in each instance, is one of aryl, aralkyl, alkaryl, cycloalkyl, heterocyclyl, heteroaryl, or -W-R⁶, where W is a chalcogen and R⁶ is alkyl;
where the ring portion of any of said aryl, aralkyl, alkaryl, cycloalkyl, heterocyclyl, or heteroaryl in R¹, R², R³ or R⁵ can be optionally substituted; and
Z¹ and Z² together form a moiety derived from a sugar, wherein the atom attached to boron in each case is an oxygen atom.

Further disclosed is a composition comprising a compound of formula (2): 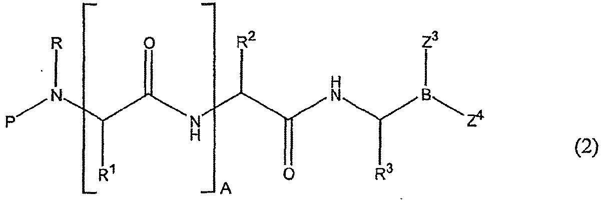 wherein:
P is hydrogen or an amino-group-protecting moiety;
R is hydrogen or alkyl;
A is 0,1, or 2;
R¹, R², and R³ are independently hydrogen, alkyl, cycloalkyl, aryl, or -CH₂-R⁵;
R⁵, in each instance, is one of aryl, aralkyl, alkaryl, cycloalkyl, heterocyclyl, heteroaryl, or -W-R⁶, where W is a chalcogen and R⁶ is alkyl;
where the ring portion of any of said aryl, aralkyl, alkaryl, cycloalkyl, heterocyclyl, or heteroaryl in R¹, R², R³ or R⁵ can be optionally substituted; and
Z³ and Z⁴ together form a moiety derived from a compound having at least two hydroxyl groups separated by at least two connecting atoms in a chain or ring, said chain or ring comprising carbon atoms and, optionally, a heteroatom or heteroatoms which can be N, S, or O;
in a lyophilized powder.

Further there is disclosed a method for formulating a boronic acid compound, the method comprising:
(a) preparing a mixture comprising
   (*i*) water,
   (*ii*) a boronic acid compound; and
   (*iii*) a compound having at least two hydroxyl groups separated by at least two connecting atoms in a chain or ring, the chain or ring comprising carbon atoms, and optionally, a heteroatom or heteroatoms which can be N, S, or O; and
(b) lyophilizing the mixture.

### DESCRIPTION OF THE PREFERRED EMBODIMENTS

Provided are specific composition as detailed in claim 2 and a method of preparing it according to claim 1.

For purposes of the present invention, the following definitions will be used:

As used herein, the terms "formulate" and "formulation" refer to the preparation of a boronic acid compound in a form suitable for administration to a mammalian subject, preferably a human. Often, formulation of the boronic acid compound comprises addition of pharmaceutically acceptable excipients, diluents, or carriers. In some embodiments, formulation of the boronic acid compound comprises formation of a chemical derivative of the boronic acid compound, preferably formation of a boronate ester. The term "formulation" refers to any form commonly used for pharmaceutical administration, including solids, liquids, suspensions, creams, and gels. For purposes of the present invention, the formulation is preferably a lyophilized powder.

As used herein, the term "lyophilized powder" refers to any solid material obtained by lyophilization of an aqueous mixture.

By "stable formulation" is meant any formulation having sufficient stability to have utility as a pharmaceutical agent. Preferably, the formulation has sufficient stability to allow storage at a convenient temperature, preferably between 0 °C and 40 °C, for a reasonable period of time, preferably longer than one month, more preferably longer than three months, even more preferably longer than six months, and most preferably longer than one year.

As employed herein, the term "boronic acid" refers to any chemical compound comprising a -B(OH), moiety. Snyder et al., J. Am. Chem. Soc. 3611 (1958), teaches that alkyl boronic acid compounds readily form oligomeric anhydrides by dehydration of the boronic acid moiety. Thus, unless otherwise apparent from context, the term "boronic acid" is expressly intended to encompass free boronic acids, oligomeric anhydrides, including, but not limited to, dimers, trimers, and tetramers, and mixtures thereof.

As employed herein, the term "compound having at least two hydroxyl groups" refers to any compound having two or more hydroxyl groups. For purposes of the present invention, the two hydroxyl groups are preferably separated by at least two connecting atoms, preferably from about 2 to about 5 connecting atoms, more preferably 2 or 3 connecting atoms. The connecting atoms may be in a chain or a ring, the chain or ring comprising carbon atoms and, optionally, a heteroatom or heteroatoms, which can be N, S, or O. For convenience, the term "dihydroxy compound" may be used to refer to a compound having at least two hydroxyl groups, as defined above. Thus, as employed herein, the term "dihydroxy compound" is not intended to be limited to compounds having only two hydroxyl groups.

As employed herein, the term "amino-group protecting moiety" refers to any group used to derivatize an amino group, especially an N-terminal amino group of a peptide or amino acid. Such groups include, without limitation, alkyl, acyl, alkoxycarbonyl, aminocarbonyl, and sulfonyl moieties. However, the term "amino-group protecting moiety" is not intended to be limited to those particular protecting groups that are commonly employed in organic synthesis, nor is it intended to be limited to groups that are readily cleavable.

The term "chalcogen" as employed herein refers to the elements oxygen or sulfur.

The term "alkyl" as employed herein refers to straight and branched chain 20 aliphatic groups having from 1 to 12 carbon atoms, preferably 1-8 carbon atoms, more preferably 1-6 carbon atoms, and still more preferably 1-4 carbon atoms, which may be optionally substituted with one, two or three substituents. Unless otherwise explicitly stated, the term "alkyl" is meant to include saturated, unsaturated, and partially unsaturated aliphatic groups. When unsaturated groups are particularly intended, the terms "alkenyl" or "alkynyl" will be used. When only saturated groups are intended, the term "saturated alkyl" will be used. Preferred saturated alkyl groups include, without limitation, methyl, ethyl, propyl, isopropyl, butyl, isobutyl, sec-butyl, *tert*-butyl, pentyl, and hexyl.

The term "cycloalkyl" as employed herein includes saturated and partially unsaturated cyclic hydrocarbon groups having 3 to 12 carbons, preferably 3 to 8 carbons, and more preferably 3 to 6 carbons, wherein the cycloalkyl group additionally may be optionally substituted. Preferred cycloalkyl groups include, without limitation, cyclopropyl, cyclobutyl, cyclopentyl, cyclopentenyl, cyclohexyl, cyclohexenyl, cycloheptyl, and cyclooctyl.

An "aryl" group is a C₆-C₁₄ aromatic moiety comprising one to three aromatic rings, which may be optionally substituted. Preferably, the aryl group is a C₆-C₁₀ aryl group. Preferred aryl groups include, without limitation, phenyl, naphthyl, anthracenyl, and fluorenyl. An "aralkyl" or "arylalkyl" group comprises an aryl group covalently linked to an alkyl group, either of which may independently be optionally substituted or unsubstituted. Preferably, the aralkyl group is (C₁-C₆)alk(C₆-C₁₀)aryl, including, without limitation, benzyl, phenethyl, and naphthylmethyl. An "alkaryl" or "alkylaryl" group is an aryl group having one or more alkyl substituents. Examples of alkaryl groups include, without limitation, tolyl, xylyl, mesityl, ethylphenyl, *tert*-butylphenyl, and methylnaphthyl.

The terms "heterocycle," "heterocyclic," and "heterocyclyl" refer to any stable ring structure having from about 3 to about 8 atoms, wherein one or more atoms are selected from the group consisting ofN, O, and S. The nitrogen and sulfur heteroatoms of the heterocyclic moiety may be optionally oxidized, and the nitrogen atoms may be optionally quaternized. The heterocyclic ring can be attached to its pendant group at any heteroatom or carbon atom that results in a stable formula. The term "stable compound" or "stable formula" is meant to refer to a compound that is sufficiently robust to survive isolation to a useful degree of purity from a reaction mixture and formulation into an efficacious therapeutic agent.

The heterocyclic group may be optionally substituted on carbon at one or more positions with any of the substituents recited above. The heterocyclic group may also independently be substituted on nitrogen with alkyl, aryl, aralkyl, alkylcarbonyl, alkylsulfonyl, arylcarbonyl, arylsulfonyl, alkoxycarbonyl, aralkoxycarbonyl, oxo, or hydroxy, or on sulfur with oxo or lower alkyl. Preferred heterocyclic groups include, without limitation, epoxy, aziridinyl, tetrahydrofuranyl, pyrrolidinyl, piperidinyl, piperazinyl, thiazolidinyl, oxazolidinyl, oxazolidinonyl, and morpholinyl. The heterocyclic group may also be fused to an aryl, heteroaryl, or heterocyclic group. Examples of such fused heterocyles include, without limitation, tetrahydroquinoline and dihydrobenzofuran.

As used herein, the terms "heteroaryl" and "aromatic heterocyle" refer to groups having 5- to 14-membered rings, preferably 5-, 6-, 9-, or 10-membered rings; having 6, 10, or 14 π electrons shared in a cyclic array; and having, in addition to carbon atoms, from one to about four, preferably from one to about three, heteroatoms selected from the group consisting of N, O, and S. The heteroaryl group may be optionally substituted on carbon at one or more positions with any of the substituents recited above. Preferred heteroaryl groups include, without limitation, thienyl, benzothienyl, furanyl, benzofuranyl, dibenzofuranyl, pyrrolyl, imidazolyl, pyrazolyl, pyridyl, pyrazinyl, pyrimidinyl, indolyl, quinolyl, isoquinolyl, quinoxalinyl, tetrazolyl, oxazolyl, thiazolyl, and isoxazolyl.

As employed herein, a "substituted" alkyl, cycloalkyl, aryl, heterocyclyl, or heteroaryl group is one having from one and to about four, preferably from one to about three, more preferably one or two, non-hydrogen substituents. Suitable substituents include, without limitation, halo, hydroxy, oxo, nitro, haloalkyl, alkyl, alkaryl, aryl, aralkyl, alkoxy, aryloxy, amino, acylamino, alkylcarbamoyl, arylcarbamoyl, aminoalkyl, alkoxycarbonyl, carboxy, hydroxyalkyl, alkylsulfonyl, arenesulfonyl, alkanesulfonamido, arenesulfonamido, aralkylsulfonamido, alkylcarbonyl, acyloxy, cyano, and ureido groups. Preferably the substituents are independently selected from the group consisting of C₁-C₆ alkyl, C₃-C₈ cycloalkyl, (C₁-C₆)alkyl(C₃-C₈)cycloalkyl, C₂-C₈ alkenyl, C₂-C₈ alkynyl, cyano, amino, C₁-C₆ alkylamino, di(C₁-C₆)alkylamino, benzylamino, dibenzylamino, nitro, carboxy, carbo(C₁-C₆)alkoxy, trifluoromethyl, halogen, C₁-C₆ alkoxy, C₆-C₁₀ aryl, (C₆-C₁₀)aryl(C₁-C₆)alkyl, (C₆-C₁₀)aryl(C₁-C₆)alkoxy, hydroxy, C₁-C₆ alkylthio, C₆-C₁₀ alkylsulfinyl, C₆-C₁₀ alkylsulfonyl, C₆-C₁₀ arylthio, C₆-C₁₀ arylsulfinyl, C₆-C₁₀ arylsulfonyl, C₆-C₁₀ aryl, (C₁-C₆)alkyl(C₆-C₁₀)aryl, and halo(C₆-C₁₀)aryl.

The term "halogen" or "halo" as employed herein refers to chlorine, bromine, fluorine, or iodine.

The term oxo refers to an oxygen atom, which forms a carbonyl when attached to carbon, an N-oxide when attached to nitrogen, and a sulfoxide or sulfone when attached to sulfur.

As herein employed, the term "acyl" refers to an alkylcarbonyl or arylcarbonyl substituent.

The term "acylamino" refers to an amide group attached at the nitrogen atom. The term "carbamoyl" refers to an amide group attached at the carbonyl carbon atom. The nitrogen atom of an acylamino or carbamoyl substituent may be additionally substituted. The term "sulfonamido" refers to a sulfonamide substituent attached by either the sulfur or the nitrogen atom. The term "amino" is meant to include NH₂, alkylamino, arylamino, and cyclic amino groups.

The term "ureido" as employed herein refers to a substituted or unsubstituted urea moiety.

In a first aspect, the invention provides compounds having formula (1): 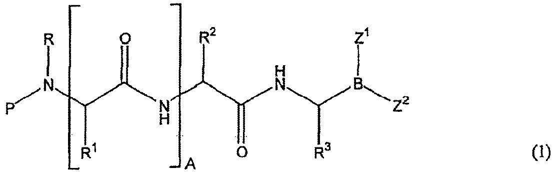 wherein
P is hydrogen or an amino-group protecting moiety;
R is hydrogen or alkyl;
A is 0, 1, or 2;
R¹, R², and R³ are independently hydrogen, alkyl, cycloalkyl, aryl, or -CH₂-R⁵;
R⁵, in each instance, is one of aryl, aralkyl, alkaryl, cycloalkyl, heterocyclyl, heteroaryl, or -W-R⁶, where W is a chalcogen and R⁶ is alkyl;
where the ring portion of any of said aryl, aralkyl, alkaryl, cycloalkyl, heterocyclyl, or heteroaryl in R¹, R², R³ or R⁵ can be optionally substituted; and
Z¹ and Z² together form a moiety derived from a sugar, wherein the atom attached to boron in each case is an oxygen atom.

As used herein, the term "moiety derived from a sugar" refers to a moiety formed by removing the hydrogen atoms from two hydroxyl groups of any sugar moiety. The moiety derived from a sugar may be attached to boron by any two of the hydroxyl groups of the sugar. For example, in various embodiments, the boronate ester forms a 5-, 6-, 7-, 8-, or 9-membered ring. In some preferred embodiments, the boronate ester forms a 5- or 6-membered ring.

The sugar is preferably a monosaccharide or disaccharide. Non-limiting examples of suitable sugars include, glucose, sucrose, fructose, trehalose, xylitol, mannitol, and sorbitol. In certain preferred embodiments, the sugar is a reduced sugar, more preferably mannitol or sorbitol. Thus, in the embodiment wherein the sugar is mannitol or sorbitol, Z¹ and Z² of the compound of formula (1) together form a moiety of formula C₆H₁₂O₆, wherein the oxygen atoms of the two deprotonated hydroxyl groups form covalent attachments with boron to form a boronate ester compound.

Preferably, the mannitol or sorbitol boronate ester compound has one of the following structures:

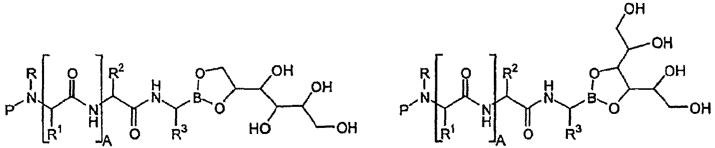

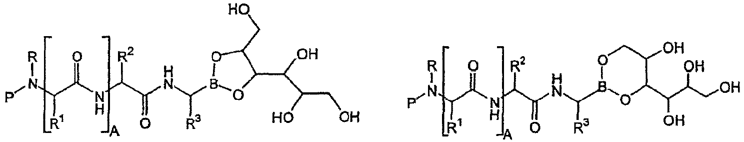

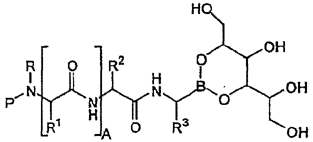

However, structures with larger boronate ester ring sizes are also possible.

In certain preferred embodiments, the mannitol or sorbitol boronate ester forms a symmetrical 5-membered ring having the following structure:

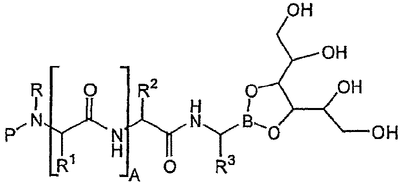

Preferably, the mannitol or sorbitol is of the D-configuration, although the L-configuration may also be used. In certain particularly preferred embodiments, Z¹ and Z² together form a moiety derived from D-mannitol. In these embodiments, the boronate ester compound preferably has one of the following structures:

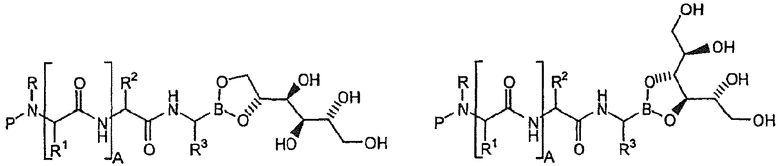

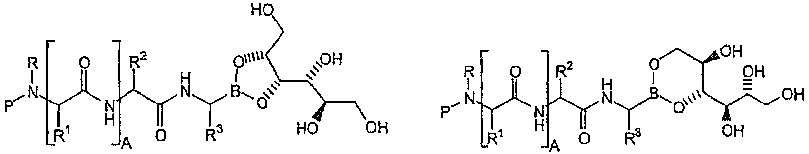

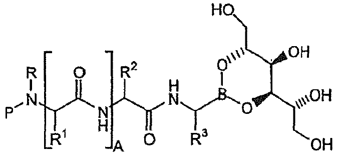

However, structures with larger boronate ester ring sizes are also possible.

In certain particularly preferred embodiments, the boronate ester compound has the following structure:

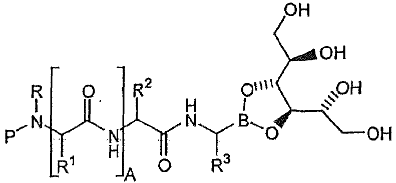

The P moiety of the compound of formula (1) is preferably hydrogen or one of R⁷-C(O)-, R⁷-S(O)-, R⁷-NH-C(O)-, or R⁷-O-C(O)-, where R⁷ is one of alkyl, aryl, alkaryl, or aralkyl, any of which can be optionally substituted, or when Y is R⁷-C(O)- or R⁷-S(O)₂-, R⁷ can also be an optionally substituted 5- to 10-membered saturated, partially unsaturated, or aromatic heterocycle.

In certain preferred embodiments, P is one of R⁷-C(O)- or R⁷-S(O)₂-, and R⁷ is an optionally substituted 5- to 10-membered saturated, partially unsaturated, or aromatic heterocycle. Preferably, R⁷ is an aromatic heterocycle, more preferably pyrazinyl, pyridyl, quinolyl, or quinoxalinyl, or a saturated heterocycle, preferably morpholinyl. In some preferred embodiments, P is (2-pyrazine)carbonyl or (2-pyrazine)sulfonyl.

In some preferred embodiments, R is hydrogen. In some other preferred embodiments, R is alkyl, preferably C₁-C₆, alkyl, more preferably C₁-C₄, alkyl, and most preferably methyl or ethyl.

The variable A in formula (1) can be 0, 1, or 2. Thus, when A is zero, the residue within the brackets is not present and the boronate ester compound is a dipeptide. Similarly, where A is 1, the residue within the brackets is present and the compound is a tripeptide. Where A is 2, the compound is a tetrapeptide. In certain particularly preferred embodiments, A is zero.

For purposes of the invention, the terms "peptide," 'dipeptide," and "tripeptide" are intended to encompass compounds comprising natural amino acid residues, unnatural amino acid residues, or a combination of natural and unnatural amino acid residues. It will be apparent from formulae (1)-(3), that the terms "peptide," "dipeptide," and "tripeptide" are used herein to refer to compounds in which the carboxylic acid functionality of the C-terminal amino acid residue is replaced by a boronic acid or boronate ester functionality.

It is preferred that the substituents R¹, R², and R³ in formula (1) are each independently one of hydrogen, C₁-C₈, alkyl, C₃-C₁₀ cycloalkyl, or C₆-C₁₀ aryl, or -CH₂-R⁵, wherein each of R¹, R², R³, and R⁵ may be optionally substituted. More preferably, R¹, R², and R³ are each independently one of C₁-C₄ alkyl or -CH₂-R⁵, and R⁵ is one of cycloalkyl, aryl, heterocyclyl, heteroaryl, or -W-R⁶ where W is chalcogen and R⁶ is alkyl. Preferably, R⁵ is one of C₆-C₁₀ aryl, (C₆-C₁₀)ar(C₁-C₆)alkyl, (C1-C₆)alk(C₆-C₁₀)aryl, C₃-C₁₀ cycloalkyl, C₁-C₈ alkoxy, or C₁-C₈, alkylthio or a 5- to 10-membered heteroaryl ring.

In certain particularly preferred embodiments, the compound of formula (1)is one of:
D-Mannitol N-(2-pyrazine)carbonyl-L-phenylalanine-L-leucine boronate;
D-Mannitol N-(2-quinoline)sulfonyl-L-homophenylalanine-L-leucine boronate;
D-Mannitol N-(3-pyridine)carbonyl-L-phenylalanine-L-leucine boronate;
D-Mannitol N-(4-morpholine)carbonyl-L-phenylalanine-L-leucine boronate;
D-Mannitol N-(4-morpholine)carbonyl-β-(1-naphthyl)-L-alanine-L-leucine boronate;
D-Mannitol N-(8-quinoline)sulfonyl-β-(1-naphthyl)-L-alanine-L-leucine boronate;
D-Mannitol N-(4-morpholine)carbonyl-(*O*-benzyl)-L-tyrosine-L-leucine boronate;
D-Mannitol N-(4-morpholine)carbonyl-L-tyrosine-L-leucine boronate; or
D-Mannitol-N-(4-morpholine)carbonyl-[*O*-(2-pyridylmethyl)]-L-tyrosine-L-leucine boronate.

In a second aspect, the invention provides a composition comprising a compound of formula (2): 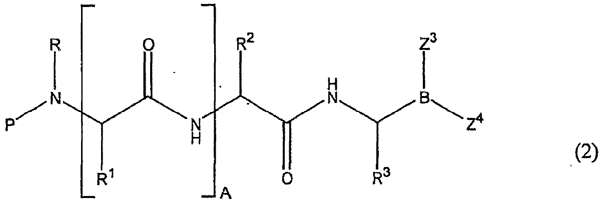 wherein:
P is hydrogen or an amino-group protecting moiety;
R is hydrogen or alkyl;
A is 0, 1, or 2;
R¹, R², and R³ are independently hydrogen, alkyl, cycloalkyl, aryl, or -CH₂-R⁵;
R⁵, in each instance, is one of aryl, aralkyl, alkaryl, cycloalkyl, heterocyclyl, heteroaryl, or -W-R⁶, where W is a chalcogen and R⁶ is alkyl;
where the ring portion of any of said aryl, aralkyl, alkaryl, cycloalkyl, heterocyclyl, or heteroaryl in R¹, R², R³ or R⁵ can be optionally substituted; and
Z³ and Z⁴ together form a moiety derived from a compound having at least two hydroxyl groups separated by at least two connecting atoms in a chain or ring, said chain or ring comprising carbon atoms and, optionally, a heteroatom or heteroatoms which can be N, S, or O; in a lyophilized powder.

Preferred values for the variables P, R, A, R¹, R², R³, R⁵, and R⁶ according to this aspect of the invention are as described above for the first aspect.

The term "moiety derived from a compound having at least two hydroxyl groups" according to this aspect of the invention is used analogously to the term "moiety derived from a sugar" described above, and thus refers to a moiety formed by removing the hydrogen atoms from two hydroxyl groups of a compound having at least two hydroxyl groups. The moiety derived from a compound having at least two hydroxyl groups may be attached to boron by the oxygen atoms of any two of its hydroxyl groups. Preferably, the boron atom, the oxygen atoms attached to boron, and the atoms connecting the two oxygen atoms together form a 5- or 6-membered ring. Examples of suitable compounds having at least two hydroxyl groups ("dihydroxy compounds") include, without limitation, pinanediol, pinacol, perfluoropinacol, ethylene glycol, diethylene glycol, catechol, 1,2-cyclohexanediol, 1,3-propanediol, 2,3-butanediol, 1,2-butanediol, 1,4-butanediol, glycerol, and diethanolamine.

For purposes of the present invention, the dihydroxy compound is D-mannitol.

The composition according to this aspect of the invention is in the form of a lyophilized, wherein the dihydroxy compound and the compound of formula (1) are present in a ratio of 10:1 w/w.

In some preferred embodiments, the composition further comprises one or more other pharmaceutically acceptable excipients, carriers, diluents fillers, salts, buffers, stabilizers, solubilizers, and other materials well known in the art. The preparation of pharmaceutically acceptable formulations containing these materials is described in, e.g., Remington's Pharmaceutical Sciences, 18th Edition, ed. A. Gennaro, Mack Publishing Co., Easton, PA, 1990.

The compounds and compositions according to the first and second aspects of the invention may be prepared by the methods described herein, or by any method suitable to produce the compound or composition. For example, the boronate esters of formula (1) can be prepared from the corresponding boronic acids by lyophilization in the presence of mannitol or sorbitol, as described herein, or, alternatively, can be prepared from another boronate ester by transesterification. Alternatively, the boronate esters of formula (1) can be prepared by incorporation of the sugar moiety at an earlier stage in the synthesis.

In a third aspect, the invention provides a method for formulating a boronic acid compound, the method comprising:
(a) preparing a mixture comprising
   *(i)* water,
   *(ii)* a boronic acid compound; and
   *(iii)* a compound having at least two hydroxyl groups separated by at least two connecting atoms in a chain or ring, the chain or ring comprising carbon atoms, and optionally, a heteroatom or heteroatoms which can be N, S, or O; and
(b) lyophilizing the mixture.

In certain preferred embodiments, the mixture comprises one or more co-solvents in addition to water. Preferably, the co-solvent is miscible with water. In the claimed invention, the aqueous solvent mixture comprises 40% v/v *tert*-butanol.

The w/w ratio of dihydroxy compound to boronic acid compound is about 10:

The aqueous mixture can be prepared by any order of addition. For example, in some embodiments, the dihydroxy compound is added to an aqueous mixture comprising a boronic acid compound. In some other embodiments, the boronic acid compound is added to an aqueous mixture comprising a dihydroxy compound. In still yet other embodiments, the boronic acid compound and dihydroxy compound can be added at the same time, or nearly at the same time. In some embodiments, it may be advantageous initially to add the boronic acid compound and/or the dihydroxy compound to a solvent mixture containing a higher percentage of co-solvent than is desired for the lyophilization step, and then dilute with water.

In some preferred embodiments, the mixture further comprises one or more pharmaceutically acceptable excipients, carriers, diluents fillers, salts, buffers, stabilizers, solubilizers, and other materials well known in the art. The preparation of pharmaceutically acceptable formulations containing these materials is described in, e.g., Remington's Pharmaceutical Sciences, 18th Edition, ed. A. Gennaro, Mack Publishing Co., Easton, PA, 1990.

Preferred compounds having at least two hydroxyl groups ("dihydroxy compounds") according to this aspect of the invention are as described above for the second aspect.

In certain preferred embodiments, the boronic acid compound has formula (3): 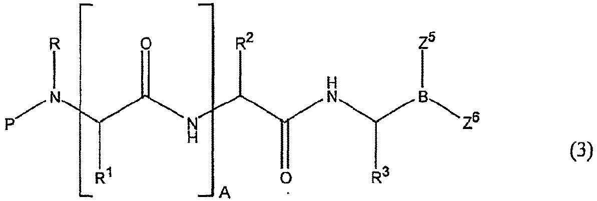 wherein:
P is hydrogen or an amino-group protecting moiety;
R is hydrogen or alkyl;
A is 0, 1, or 2;
R¹, R², and R³ are independently hydrogen, alkyl, cycloalkyl, aryl, or - CH₂-R⁵;
R⁵, in each instance, is one of aryl, aralkyl, alkaryl, cycloalkyl, heterocyclyl, heteroaryl, or -W-R⁶, where W is a chalcogen and R⁶ is alkyl;
where the ring portion of any of said aryl, aralkyl, alkaryl, cycloalkyl, heterocyclyl, or heteroaryl in R¹, R², R³ or R⁵ can be optionally substituted; and
Z⁵ and Z⁶ are each OH.

Preferred values for the variables P, R, A, R¹, R², R³, R⁵, and R⁶ according to this aspect of the invention are as described above for the first aspect.

In certain particularly preferred embodiments, the boronic acid compound is one of:
N-(2-pyrazine)carbonyl-L-phenylalanine-L-leucine boronic acid;
N-(2-quinoline)sulfonyl-L-homophenylalanine-L-leucine boronic acid;
N-(3-pyridine)carbonyl-L-phenylalanine-L-leucine boronic acid;
N-(4-morpholine)carbonyl-L-phenylalanine-L-leucine boronic acid;
N-(4-morpholine)carbonyl-β-(1-naphthyl)-L-alanine-L-leucine boronic acid;
N-(8-quinoline)sulfonyl-β-(1-naphthyl)-L-alanine-L-leucine boronic acid;
N-(4-morpholine)carbonyl-(*O*-benzyl)-L-tyrosine-L-leucine boronic acid;
N-(4-morpholine)carbonyl-L-tyrosine-L-leucine boronic acid; or
N-(4-morpholine)carbonyl-[*O*-(2-pyridylmethyl)]-L-tyrosine-L-leucine boronic acid.

In a fourth aspect, the invention provides compositions prepared according to the methods according to the third aspect of the invention. In some preferred embodiments, formulation of a boronic acid according to the methods of the invention results in formation of a chemical derivative of the boronic acid compound, preferably formation of a boronate ester. In these embodiments, formulation of a boronic acid compound according to the method of the invention produces a composition comprising a boronate ester compound, according to the second aspect of the invention.

In some other embodiments, formulation of a boronic acid compound according to the method of the invention does not result in formation of a chemical derivative of the boronic acid compound. In these embodiments, the composition according to the third aspect of the invention comprises a boronic acid compound and a compound having at least two hydroxyl groups in a lyophilized powder.

The compositions according to the second and fourth aspects of the invention can be readily reconstituted by adding an aqueous solvent. Preferably, the reconstitution solvent is suitable for pharmaceutical administration. Examples of suitable reconstitution solvents include, without limitation, water, saline, and phosphate buffered saline (PBS). For clinical use, the compositions according to the second aspect of the invention are preferably reconstituted with sterile saline (0.9% w/v).

Upon reconstitution in aqueous medium, an equilibrium is established between any boronate ester present in the composition and the corresponding boronic acid. Typically, equilibrium is reached quickly, e.g., within 10-15 minutes, after the addition of water. The relative concentrations of boronate ester and boronic acid present at equilibrium is dependent upon the pH of the solution, temperature, and the ratio of dihydroxy compound to boronic acid compound.

The following examples are intended to further illustrate certain preferred embodiments of the invention, and are not intended to limit the scope of the invention.

### EXAMPLES

### Example 1: Preparation of a lyophilized formulation of N-(2-pyrazine)carbonyl-L-phenylalanine-L-leucine boronic acid with D-mannitol

Approximately 40 mg of N-(2-pyrazine)carbonyl-L-phenylalanine-L-leucine boronic acid was weighed into a container, and 16 mL of *tert-*butanol was added. The container was closed and the suspension was warmed to approximately 45 °C for 5 minutes to complete dissolution of the compound. Water (24 mL) was added with stirring, followed by 0.4 g of mannitol, added as an excipient, 1% w/v. The mixture was stirred to complete dissolution and then cooled to ambient temperature. The solution ' was filtered through a 0.45 µm nylon membrane. One milliliter aliquots were placed in 5 mL serum bottles. Split rubber stoppers were partially inserted into the bottles, and the bottles were placed in a freeze dryer with a shelf temperature of -45 °C. After approximately 1 hour, the vacuum was applied. The shelf temperature was allowed to rise gradually to -35 °C and maintained at -35 °C until the ice was gone from the samples (approximately 40 hours). The shelf temperature control was then turned off and the shelf temperature was allowed to gradually rise to 0 °C. A secondary drying cycle was carried out by increasing the shelf temperature in 3 increments to 25 °C over a time period of 1.5 hours. The shelf temperature was maintained at 25 °C for 2 hours. The samples were sealed under nitrogen and removed from the freeze dryer.

The residual moisture content of the samples was determined by Karl Fischer analysis, using three lyophilized products. The water content was 0.88% by weight.

Fast Atom Bombardment (FAB) mass spectral analysis of the lyophilized product showed a strong signal at m/z =531, indicative of formation of a covalent boronate ester adduct between N-(2-pyrazine)carbonyl-L-phenylalanine-L-leucine boronic acid and D-mannitol. Glycerol was employed as the matrix, and a signal for the glycerol adduct with N-(2-pyrazine)carbonyl-L-phenylalanine-L-leucine boronic acid was observed at *m*/*z* = 441. However, the intensity of the signal at m/z=441 was very low compared to the signal at m/z =531, possibly indicative of the enhanced stability of the D-mannitol adduct.

### Example 2: Production-scale preparation of a lyophilized formulation of N-(2-pyrazine)carbonyl-L-phenylalanine-L-leucine boronic acid with D-mannitol

In a clean compounding vessel, a solution of 97% tert-butanol/3% Water for Injection was prepared by warming the required amount of *tert*-butanol to 35 °C and adding Water for Injection. Approximately 5% of the solution was reserved for use in rinsing. The solution was cooled to 15-30 °C, and *N*-(2-pyrazine)carbonyl-L-phenylalanine-L-leucine boronic acid was added with stirring. The container was rinsed with the reserved *tert*-butanol/water solution, and the rinses were added to the main vessel. The mixture was stirred until the boronic acid compound was completely dissolved. Mannitol was added, with residual mannitol being rinsed into the reaction vessel with fresh Water for Injection. Sufficient Water for Injection was added to reduce the total alcohol content to 40% v/v. The mixture was stirred until the mannitol was completely dissolved. The mixture was filtered through a 0.22 micron filter. Aliquots of the filtered solution were placed into previously sterilized vials. The vials were sealed with lyophilization stoppers and were placed on lyophilizer chamber shelves maintained at -45 °C. After two hours, the freeze dryer chamber was evacuated and the chamber pressure was adjusted to 100-200 microns with sterile nitrogen. The lyophilizer chamber shelves were warmed to -30 °C using an appropriate ramp rate, and held at that temperature for 10-15 hours. After each of the product thermocouples read - 33 °C or warmer, the shelf temperature was adjusted to -15 °C over 7 hours using an appropriate ramp rate and maintained at that temperature for 5 hours. After all product thermocouples recorded the shelf temperature, the shelf was warmed to 0 °C over a period of at least 7 hours using an appropriate ramp rate. When all thermocouples recorded 0 °C, the shelf was warmed to 27 °C and maintained at that temperature for 4 hours. At the end of the terminal drying phase, the chamber pressure was restored using sterile nitrogen, and the vials were sealed and removed.

### Example 3; Reconstitution of N-(2-pyrazine)carbonyl-L-phenylalanine-L-leucine boronic acid

The lyophilized formulation of *N*-(2-pyrazine)carbonyl-L-phenylalanine-L-leucine boronic acid with D-mannitol was prepared as described in Example 1. One sample was reconstituted with 2 mL of water. Dissolution was complete within 1-2 minutes of shaking. The entire solution was transferred to a volumetric flask, diluted, and analyzed by HPLC for content of N-(2-pyrazine)carbonyl-L-phenylalanine-L-leucine boronic acid. The total drug content was 1.09 mg. A second sample was reconstituted with 1 mL of propylene glycol:EtOH:H₂O, 40:10:50. Dissolution was complete with 1 minute of shaking. The total drug content was 1.11 mg.

The lyophilized formulation was also reconstituted with 0.9% w/v saline. The lyophilized material dissolved readily at concentrations up to 6 mg/mL. By contrast, solid *N*-(2-pyrazine)carbonyl-L-phenylalanine-L-leucine boronic acid was not soluble in 0.9% w/v saline at a concentration of 1 mg/mL.

To be certain that free N-(2-pyrazine)carbonyl-L-phenylalanine-L-leucine boronic acid was rapidly liberated upon reconstitution of the lyophilized formulation in aqueous solution, the lyophilized formulation was dissolved in neat DMSO and assayed for inhibition of the chymotrypsin-like activity of the 20S proteasome as described in U.S. Pat. No. 5,780,454. Proteasome inhibition can only be observed if hydrolysis under the assay conditions is fast. The observed Kᵢ value of 0.3 nM is equivalent to that observed for free N-(2-pyrazine)carbonyl-L-phenylalanine-L-leucine boronic acid, indicating complete and rapid hydrolysis of the D-mannitol adduct under the assay conditions.

### Example 4: HPLC Analysis of N-(2-pyrazine)carbonyl-L-phenylalanine-L-leucine boronic acid

### System Parameters:

| | | |
|---|---|---|
| *Column.* | | Adsorbosphere-HS-C18, 5µ, 250 x 4.6 mm |
| | *Mobile Phase:* | 65/35: methanol/water containing 0.1% TFA |
| | *Flow Rate:* | 1.0 mL/min |
| | *Detection*/*Sensitivity:* | PDA and UV at 255 nm, 0.1 aufs |
| | *Injection volume:* | 25 µL |
| Internal Standard Solution: | | 0.18 mg/mL diphenylamine in methanol |
| Sample Preparation: | | Accurately weighed 0.5-1.5 mg portions of the sample or reference standard were dissolved in 2.00 mL of the internal standard solution. |

### Chromatographic parameters:

| | **Sample** | **Internal Standard** |
|---|---|---|
| *Retention time* | 8.4 min | 18.9 min |
| *Capacity factor, k'* | 2.0 | 5.8 |
| *Asymmetry (10%)* | 1.7 | 1.3 |
| *Rel. Retention,* α | 0.34 | |
| *Resolution, Rₛ* = *ΔT*/*∑W*_{*1*/*2*} | 15.1 | |

ΔT and ∑W_{1/2} are, respectively, the differences in retention times and the sum of the mid-width of the sample and internal standard peaks. Minor variation of the mobile phase is allowed to achieve results similar to those above.

### Example 5: Stability of formulations

### Solid N-(2-pyrazine)carbonyl-L-phenylalanine-L-leucine boronic acid

*N*-(2-pyrazine)carbonyl-L-phenylalanine-L-leucine boronic acid was prepared as described in U.S. Pat. No. 5,780,454. The product was obtained as a white amorphous powder. The product was stable for more than 2 years when stored at -20 °C, as determined by HPLC analysis (purity >97%). When stored at 2-8 °C, the product was not stable for longer than 3-6 months.

### Liquid N-(2-pyrazine)carbonyl-L-phenylalanine-L-leucine boronic acid

A sterile liquid formulation (0.5 mg/mL) of *N*-(2-pyrazine)carbonyl-L-phenylalanine-L-leucine boronic acid was prepared in 0.9% w/v saline, 2% v/v ethanol and 0.1% w/v ascorbic acid. When stored at 2-8 °C, the liquid formulation was not stable for longer than 6 months, as determined by HPLC analysis.

### Lyophilized D-mannitol N-(2-pyrazine)carbonyl-L-phenylalanine-L-leucine boronate

The lyophilized product was prepared according to Example 1 and stored at 5 °C, ambient temperature, 37 °C, and 50 °C. Stability was monitored for approximately 18 months by periodically reconstituting a sample and analyzing the entire contents of the bottle by HPLC. Over this time period, there was no loss of drug in the lyophilized product stored at any temperature and no evidence of degradation product peaks in the HPLC chromatograms.

### Reconstituted solution of N-(2-pyrazine)carbonyl-L-phenylalanine-L-leucine boronic acid

The lyophilized product was prepared according to Example 1, and samples (2.5 mg/vial) were reconstituted with 2.5 mL of 0.9% w/v sterile saline: Dissolution was complete within 10 seconds and afforded a clear, colorless solution containing 1 mg/mL of *N*-(2-pyrazine)carbonyl-L-phenylalanine-L-leucine boronic acid. The solution showed no sign of degradation when stored at ambient temperature (23 °C) for 43 hours. No special care was taken to protect the solution from light.

## Claims

1. A method of preparing a lyophilized compound of the formula (1): 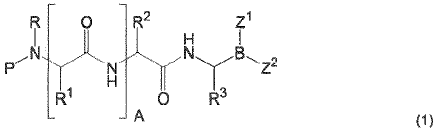 wherein
P is hydrogen or an amino-group protecting moiety;
R is hydrogen or alkyl;
A is 0, 1, or 2;
R¹, R², and R³ are each independently hydrogen, alkyl, cycloalkyl, aryl, or -CH₂-R⁵; R⁵ in each instance is aryl, aralkyl, alkaryl, cycloalkyl, heterocyclyl, heteroaryl, or -W-R⁸, where W is a chalcogen and R⁶ is alkyl;
wherein the ring portion of any said aryl, aralkyl, alkyaryl, cycloalkyl, heterocyclyl, or heteroaryl in R¹, R², R³, or R⁵ can be optionally substituted; and
Z¹ and Z² together form a moiety derived from D-mannitol;
the method comprising:
(a) preparing a mixture comprising
(i) water,
(ii) a compound of formula (3) 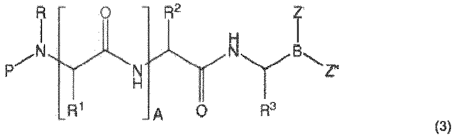 wherein P, R, A, R¹, R², and R³ are as described above; and Z' and Z" are OH;
(iii) D-mannitol; and
(iv) tert-butanol;
wherein the D-mannitol and the compound of formula (3) are present in a w/w ratio of 10:1; and wherein the aqueous solvent mixture comprises 40% *v*/*v tert*-butanol; and
(b) lyophilizing the mixture;
wherein:
the compound of formula (1) is D-mannitol *N*-(2-pyrazine)carbonyl-L-phenylalanine-L-leucine boronate; and
the compound of formula (3) is *N*-(2-pyrazine)carbonyl-L-phenylalanine-L-leucine boronic acid.

2. A lyophilized composition comprising a compound of the formula (1): 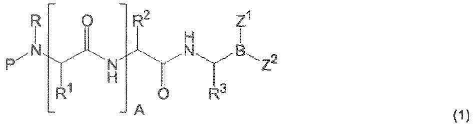 wherein
P is hydrogen or an amino-group protecting moiety;
R is hydrogen or alkyl;
A is 0, 1, or 2;
R¹, R², and R³ are each independently hydrogen, alkyl, cycloalkyl, aryl, or -CH₂-R⁵;
R⁵ in each instance is aryl, aralkyl, alkaryl, cycloalkyl, heterocyclyl, heteroaryl, or -W-R⁶, where W is a chalcogen and R⁶ is alkyl;
wherein the ring portion of any said aryl, aralkyl, alkyaryl, cycloalkyl, heterocyclyl, or heteroaryl in R¹, R², R³, or R⁵ can be optionally substituted; and
Z¹ and Z² together form a moiety derived from D-mannitol;
wherein the lyophilized composition Is prepared by a method comprising:
(a) preparing a mixture comprising
(i) water,
(ii) a compound of formula (3) 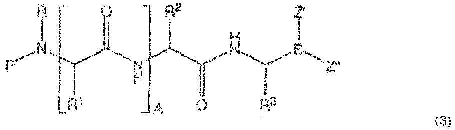 wherein P, R, A, R¹, R², and R³ are as described above; and Z' and Z" are OH;
(iii) D-mannitol; and
(iv) *tert*-butanol;
wherein the D-mannitol and the compound of formula (3) are present in a w/w ratio of 10:1; and wherein the aqueous solvent mixture comprises 40% v/v *tert*-butanol; and
(b) lyophilizing the mixture;
wherein:
the compound of formula (1) is D-mannitol *N*-(2-pyrazine)carbonyl-L-phenylalanine-L-leucine boronate; and
the compound of formula (3) is *N*-(2-pyrazine)carbonyl-L-phenylalanine-L-leucine boronic acid.

## Patentansprüche

1. Verfahren zur Herstellung einer lyophilisierten Verbindung von Formel (1): 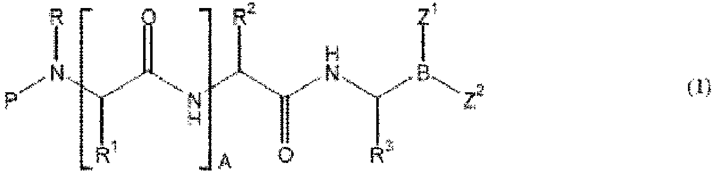 wobei
P Wasserstoff oder ein Aminogruppenschutzanteil ist;
R Wasserstoff oder Alkyl ist;
A 0, 1 oder 2 ist;
R¹, R² und R³ jeweils unabhängig Wasserstoff, Alkyl, Cycloalkyl, Aryl oder -CH₂-R⁵ sind;
R⁵ in jedem Fall Aryl, Aralkyl, Alkaryl, Cycloalkyl, Heterocyclyl, Heteroaryl oder -W-R⁶ ist, wobei W ein Chalcogen ist und R⁶ Alkyl ist;
wobei der Ringteil jedes genannten Aryls, Aralkyls, Alkyaryls, Cycloalkyls, Heterocyclyls oder Heteroaryls in R¹, R², R³ oder R⁵ optional substituiert sein kann; und Z¹ und Z² zusammen einen von D-Mannitol abgeleiteten Anteil bilden;
wobei das Verfahren Folgendes beinhaltet:
(a) Herstellen eines Gemischs, das Folgendes beinhaltet:
(i) Wasser,
(ii) eine Verbindung von Formel (3) 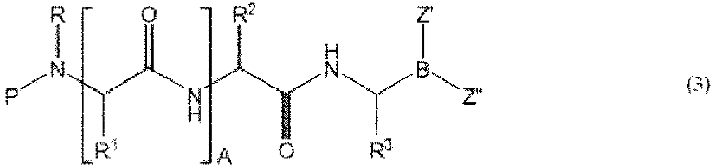 wobei P, R, A, R¹, R² und R³ wie oben beschrieben sind; und Z' und Z" OH sind;
(iii) D-Mannitol; und
(iv) *tert*-Butanol;
wobei das D-Mannitol und die Verbindung von Formel (3) in einem Gew.-Verhältnis von 10:1 vorliegen; und wobei das wässrige Lösungsmittelgemisch 40 Vol.-% tert-Butanol beinhaltet; und
(b) Lyophilisieren des Gemischs;
wobei:
die Verbindung von Formel (1) D-Mannitol-*N*-(2-pyrazin)carbonyl-L-phenylalanin-L-leucinboronat ist; und
die Verbindung von Formel (3) *N*-(2-Pyrazin)carbonyl-L-phenylalanin-L-leucinboronsäure ist.

2. Lyophilisierte Zusammensetzung, die eine Verbindung von Formel (1) beinhaltet: 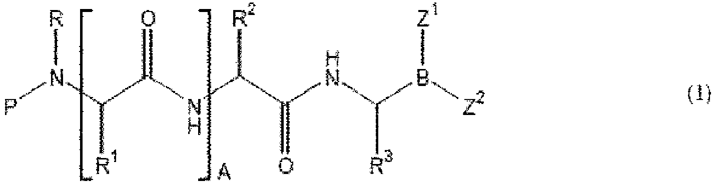 wobei
P Wasserstoff oder ein Aminogruppenschutzanteil ist;
R Wasserstoff oder Alkyl ist;
A 0, 1 oder 2 ist;
R¹, R² und R³ jeweils unabhängig Wasserstoff, Alkyl, Cycloalkyl, Aryl oder -CH₂-R⁵ sind;
R⁵ in jedem Fall Aryl, Aralkyl, Alkaryl, Cycloalkyl, Heterocyclyl, Heteroaryl oder -W-R⁶ ist, wobei W ein Chalcogen ist und R⁶ Alkyl ist;
wobei der Ringteil jedes genannten Aryls, Aralkyls, Alkyaryls, Cycloalkyls, Heterocyclyls oder Heteroaryls in R¹, R², R³ oder R⁵ optional substituiert sein kann; und Z¹ und Z² zusammen einen von D-Mannitol abgeleiteten Anteil bilden;
wobei die lyophilisierte Zusammensetzung mit einem Verfahren hergestellt wird, das Folgendes beinhaltet:
(a) Herstellen eines Gemischs, das Folgendes beinhaltet:
(i) Wasser,
(ii) eine Verbindung von Formel (3) 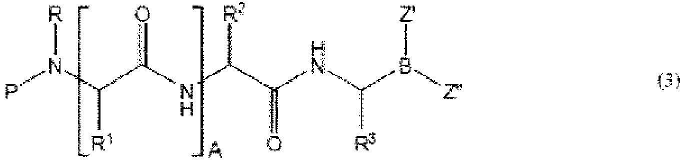 wobei P, R, A, R¹, R² und R³ wie oben beschrieben sind; und Z' und Z" OH sind;
(iii) D-Mannitol; und
(iv) tert-Butanol;
wobei das D-Mannitol und die Verbindung von Formel (3) in einem Gew.-Verhältnis von 10:1 vorliegen; und wobei das wässrige Lösungsmittelgemisch 40 Vol.-% tert-Butanol beinhaltet; und
(b) Lyophilisieren des Gemischs;
wobei:
die Verbindung von Formel (1) D-Mannitol-*N*-(2-pyrazin)carbonyl-L-phenylalanin-L-leucinboronat ist; und
die Verbindung von Formel (3) *N*-(2-Pyrazin)carbonyl-L-phenylalanin-L-leucinboronsäure ist.

## Revendications

1. Procédé de préparation d'un composé lyophilisé ayant la formule (1) : 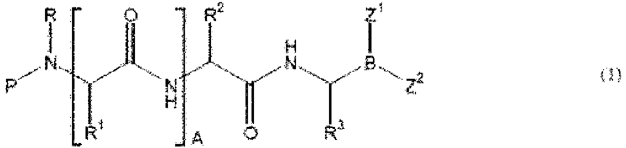 dans laquelle
P est un atome d'hydrogène ou une fraction protégeant un groupe amino ;
R est un atome d'hydrogène ou un groupe alkyle ;
A vaut 0, 1 ou 2 ;
R¹, R², et R³ sont chacun indépendamment un atome d'hydrogène, un groupe alkyle, un groupe cycloalkyle, un groupe aryle, ou un groupe -CH₂-R⁵ ;
R⁵, dans chaque cas, est un groupe aryle, aralkyle, alkaryle, cycloalkyle, hétérocyclyle, hétéroaryle, ou - W-R⁶, dans lequel W est un chalcogène et R⁶ est un groupe alkyle ;
la partie cyclique de n'importe lequel desdits groupes aryle, aralkyle, alkyaryle, cycloalkyle, hétérocyclyle, ou hétéroaryle dans R¹, R², R³, ou R⁵ pouvant optionnellement être substituée ; et
Z¹ et Z², ensemble, forment une fraction dérivée de D-mannitol ;
le procédé comprenant :
(a) la préparation d'un mélange comprenant :
(i) de l'eau,
(ii) un composé ayant la formule (3) 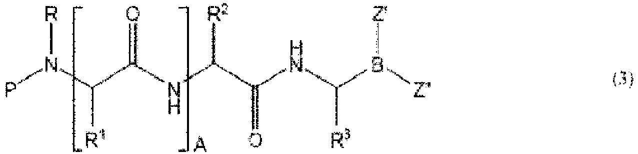 dans laquelle P, R, A, R¹, R², et R³ sont tels que décrits ci-dessus ; et Z' et Z" sont un groupe OH ;
(iii) du D-mannitol ; et
(iv) du tert-butanol ;
dans lequel le D-mannitol et le composé ayant la formule (3) sont présents selon un rapport de 10:1 p/p ; et dans lequel le mélange de solvants aqueux comprend 40 % v/v de tert-butanol ; et
(b) la lyophilisation du mélange ;
dans lequel :
le composé ayant la formule (1) est le N-(2-pyrazine)carbonyl-L-phénylalanine-L-leucine boronate de D-mannitol ; et
le composé ayant la formule (3) est l'acide N-(2-pyrazine)carbonyl-L-phénylalanine-L-leucine boronique.

2. Composition lyophilisée comprenant un composé ayant la formule (1) : 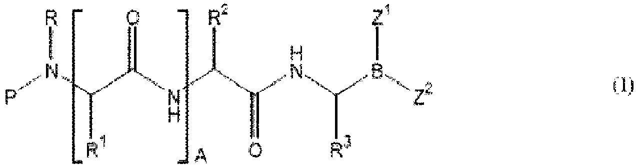 dans laquelle
P est un atome d'hydrogène ou une fraction protégeant un groupe amino ;
R est un atome d'hydrogène ou un groupe alkyle ;
A vaut 0, 1 ou 2 ;
R¹, R², et R³ sont chacun indépendamment un atome d'hydrogène, un groupe alkyle, un groupe cycloalkyle, un groupe aryle, ou un groupe -CH₂-R⁵ ;
R⁵, dans chaque cas, est un groupe aryle, aralkyle, alkaryle, cycloalkyle, hétérocyclyle, hétéroaryle, ou - W-R⁶, dans lequel W est un chalcogène et R⁶ est un groupe alkyle ;
la partie cyclique de n'importe lequel desdits groupes aryle, aralkyle, alkyaryle, cycloalkyle, hétérocyclyle, ou hétéroaryle dans R¹, R², R³, ou R⁵ pouvant optionnellement être substituée ; et
Z¹ et Z², ensemble, forment une fraction dérivée de D-mannitol ;
la composition lyophilisée étant préparée par un procédé comprenant :
(a) la préparation d'un mélange comprenant :
(i) de l'eau,
(ii) un composé ayant la formule (3) 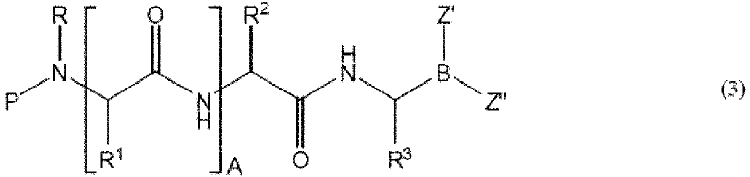 dans laquelle P, R, A, R¹, R², et R³ sont tels que décrits ci-dessus ; et Z' et Z" sont un groupe OH ;
(iii) du D-mannitol ; et
(iv) du *tert*-butanol ;
dans lequel le D-mannitol et le composé ayant la formule (3) sont présents selon un rapport de 10:1 p/p ; et dans lequel le mélange de solvants aqueux comprend 40 % v/v de *tert*-butanol ; et
(b) la lyophilisation du mélange ;
dans lequel :
le composé ayant la formule (1) est le *N-(2-*pyrazine)carbonyl-L-phénylalanine-L-leucine boronate de D-mannitol ; et
le composé ayant la formule (3) est l'acide *N*-(2-pyrazine)carbonyl-L-phénylalanine-L-leucine boronique.
